# EUROPEAN PATENT SPECIFICATION

(11) **EP 1 917 978 B1**
(45) Date of publication and mention of the grant of the patent: **20.08.2014**
(21) Application number: 06781853.4
(22) Date of filing: 21.07.2006
(51) Int. Cl.: A61K 31/519, A61K 9/20, A61P 15/08, A61P 43/00

(54) **Premature ovulation preventive agent**
Mittel zur Verhinderung vorzeitiger Ovulation
Agent préventif de l'ovulation prématurée

(30) Priority: 22.07.2005 JP 2005212973
(43) Date of publication of application: 07.05.2008
(73) Proprietor: Takeda Pharmaceutical Company Limited, Osaka-shi, Osaka 541-0045 (JP)
(72) Inventor: FURUYA, Shuichi, Tsukuba-shi, Ibaraki 300-4293 (JP); KUSAKA, Masami, Tsukuba-shi, Ibaraki 300-4293 (JP)
(74) Representative: Kingsbury, Oliver William
(86) International application number: PCT/JP2006/314943
(87) International publication number: WO 2007/011072

(56) References cited:
- WO-A1-00/56739
- WO-A1-00/59542
- WO-A1-2004/067535
- JP-A- 2001 278 884
- JP-A- 2004 250 439
- SASAKI S ET AL: "Discovery of a thieno(2,3-d) pyrimidine-2,4-dione bearing a P-methoxyureidophenyl moiety at the 6-position: ....... a antagonist for the human lutenizing" JOURNAL OF MEDICINAL CHEMISTRY, AMERICAN CHEMICAL SOCIETY, WASHINGTON, US LNKD- DOI:10.1021/JM020180I, vol. 46, no. 1, 2 January 2003 (2003-01-02), pages 113-124, XP002967759 ISSN: 0022-2623
- HARA T ET AL: "Suppression of a Pituitary-Ovarian Axis by Chronic Oral Administration of a Novel Nonpeptide Gonadotropin-Releasing Hormone Antagonist, TAK-013, in Cynomolgus Monkeys" JOURNAL OF CLINICAL ENDOCRINOLOGY & METABOLISM, vol. 88, no. 4, 1 April 2003 (2003-04-01), pages 1697-1704, XP002979733 ISSN: 0013-7227
- COCCIA M.E. ET AL.: 'GnRH antagonists' EUROPEAN JOURNAL OF OBSTETRICS & GYNECOLOGY AND REPRODUCTIVE BIOLOGY vol. 115, no. SUPPLEMENT 1, 2004, pages S44 - S56, XP003002720
- 'Final Draft Cetrotide', [Online] 2000, XP003002721 Retrieved from the Internet: <URL:http://www.fda.gov/cder/foi/label/2000 /211971b1.pdf#search=%22Cetrotide%20%22>

## Description

### Technical Field

The present invention relates to a pharmaceutical use of a nonpeptidic compound having a gonadotropin releasing hormone antagonistic action, and specifically relates to a premature ovulation inhibitor for use in in vitro fertilization or embryo transfer process.

### Background of the Invention

Secretion of anterior pituitary hormone is regulated by peripheral hormones secreted from respective hormone target organs and releasing or inhibiting hormone (hereinafter these hormone groups are generically referred to as hypothalamic hormones in the present specification) secreted from hypothalamus, which is the upper nerve of anterior pituitary. Heretofore, 9 kinds of hormones have been confirmed to be present as hypothalamic hormones, for example, thyrotropin releasing hormone (TRH), gonadotropin releasing hormone {GnRH: also referred to as luteinizing hormone releasing hormone (LH-RH)}. These hypothalamic hormones are assumed to express their hormone action via receptors considered to be present in the anterior pituitary, and analyses of receptor genes, including those of human, specific to these hormones are ongoing. Accordingly, an antagonist or agonist specific and selective to those receptors will control action of hypothalamic hormones and regulate secretion of anterior pituitary hormones. Consequently, the antagonist or agonist is expected to prevent or treat diseases dependent on those anterior pituitary hormones.

WO00/56739 and WO04/067535 disclose that a gonadotropin releasing hormone (GnRH) antagonist is useful as an agent for the prophylaxis or treatment of sex hormone dependent cancers (e.g., prostate cancer, uterine cancer, breast cancer, pituitary tumor, etc.), prostatic hyperplasia, uterine fibroid, endometriosis, precocious puberty, amenorrhea, premenstrual syndrome, multilocular ovary syndrome, acne and the like; a reproduction regulator for males and females (e.g., birth control agent, menstrual cycle regulator, etc.); a contraceptive drug for males and famales; an ovulation inducing agent for females; an infertility therapy agent; and as an estrus regulator for animals, a meat quality improver for edible meat, and a growth promoter for animals in the animal husbandry field.

For infertility treatment, use of in vitro fertilization is on the rise. It is used for infertile patients with fallopian tube disease, endemetriosis, oligospermia, antisperm antibody, and other infertility of unknown cause.

In in vitro fertilization a treatment for stimulating the ovary such as a combined use of clomiphene citrate and HMG, (Human Menopausal Gonadotrophin), single administration of HMG, a combined use of a GnRH agonist and HMG, and the like is performed to mature the occyte. Generally, after suitable maturation of the ovarian follicle an oocyte is transvaginally collected under ultrasound supervision, and the collected oocyte is subjected to in vitro fertilization. An incubated embryo is transferrer into the uterine cavity and, when successful, implanted and results in pregnancy. For infertility treatment, in vitro fertilization where ovum collection to embryo transfer are performed in a sequence of procedures, as well as frozen embryo transfer and gamete intrafallopian transfer.

Use of a peptidic GnRH antagonist that lowers the LH (Luteinizing hormone) level in in vitro fertilization (IVF) has been reported (European journal of Obstetrics & Gynecology and Reproductive Biology 115S (2004) S44-S56).

However, peptidic compounds are still problematic in many respects such as oral absorbability administration form, dose, stability of pharmaceutical agent, sustainability of action and metabolic stability. There is a strung demand for a nonpeptidic compound having a gonadotropin releasing hormone antagonistic action, which is superior in oral absorbability, can enhance or assist in vitro fertilization, and does not cause a transient pituitary gonadotropin action (acute action).

An object of the present invention is to provide a premature ovulation inhibitor for use in in vitro fertilization or embryo transfer process, which has superior GnRH antagonistic action, is low toxic, superior in oral absorbability, sustainability of action, stability and pharmacokinetics, easy to produce, and can be safely used for enhancing or assisting in vitro fertilization or embryo transfer process.

### Disclosure of the Invention

The present inventors have unexpectedly found that a compound having a nonpeptidic gonadotropin releasing hormone antagonistic action is, based on its specific chemical structure, useful as a premature ovulation inhibitor for in vitro fertilization or embryo transfer process, or a premature ovulation inhibitor under controlled ovarian stimulation, and can be administered orally. Further studies based on these findings resulted in the completion of the present invention.

Accordingly, the present invention relates to [1] A compound, N-(4-(1-(2,6-difluorobenzyl)-5-((dimethylamino)methyl)-3-(6-methoxy-3-pyridazinyl)-2,4-dioxo-1,2,3,4-tetrahydrothieno[2,3-d]pyrimidin-6-yl)phenyl)-N'-methoxyurea or a salt thereof, for use in inhibiting premature ovulation during in vitro fertilization or embryo transfer process in a mammal, excluding industrial or commercial human embryo transfer processes.

As the salt of N-(4-(1-(2,6-difluorobenzyl)-5-((dimethylamino)methyl)-3-(6-methoxy-3-pyridazinyl)-2,4-dioxo-1,2,3,4-tetrahydrothieno[2,3-d]pyrimidin-6-yl)phenyl)-N'-methoxyurea, physiologically acceptable acid addition salts are preferable. Examples of such salt include salts with inorganic acid (e.g., hydrochloric acid, hydrobromic acid, nitric acid, sulfuric acid and phosphoric acid) and salts with organic acid (e.g., formic acid, acetic acid, trifluoroacetic acid, fumaric acid, oxalic acid, tartaric acid, maleic acid, citric acid, succinic acid, malic acid, methanesulfonic acid, benzenesulfonic acid and p-toluenesulfonic acid).

N-(4-(1-(2,6-difluorobenzyl)-5-((dimethylamino)methyl)-3-(6-methoxy-3-pyridazinyl)-2,4-dioxo-1,2,3,4-tetrahydrothieno[2,3-d]pyrimidin-6-yl)phenyl)-N'-methoxyurea can be produced by a known method, for example, the methods described in WO04/067535.

When N-(4-(1-(2,6-difluorobenzyl)-5-((dimethylamino)methyl)-3-(6-methoxy-3-pyridazinyl)-2,4-dioxo-1,2,3,4-tetrahydrothieno[2,3-d]pyrimidin-6-yl)phenyl)-N'-methoxyurea has one or more asymmetric carbons in a molecule, both R-configuration and S-configuration due to such asymmetric carbon are also encompassed in the present invention.

N-(4-(1-(2,6-difluorobenzyl)-5-((dimethylamino)methyl)-3-(6-methoxy-3-pyridazinyl)-2,4-dioxo-1,2,3,4-tetrahydrothieno[2,3-d]pyrimidin-6-yl)phenyl)-N'-methoxyurea may be labeled with an isotope (e.g., ³H, ¹⁴C, ³⁵S).

When N-(4-(1-(2,6-difluorobenzyl)-5-((dimethylamino)methyl)-3-(6-methoxy-3-pyridazinyl)-2,4-dioxo-1,2,3,4-tetrahydrothieno[2,3-d]pyrimidin-6-yl)phenyl)-N'-methoxyurea has isomers such as tautomers, optical isomers, stereoisomers, positional isomers and rotational isomers, any isomers and a mixture of isomers are also encompassed in the compounds of the present invention.

Furthermore, when N-(4-(1-(2,6-difluorobenzyl)-5-((dimethylamino)methyl)-3-(6-methoxy-3-pyridazinyl)-2,4-dioxo-1,2,3,4-tetrahydrothieno[2,3-d]pyrimidin-6-yl)phenyl)-N'-methoxyurea has optical isomers, those resolved from the racemates are also encompassed in the compound of the present invention. These isomers can be respectively obtained as single products according to a synthesis method or separation method known *per se* (concentration, solvent extraction, column chromatography and recrystallization).

N-(4-(1-(2,6-difluorobenzyl)-5-((dimethylamino)methyl)-3-(6-methoxy-3-pyridazinyl)-2,4-dioxo-1,2,3,4-tetrahydrothieno[2,3-d]pyrimidin-6-yl)phenyl)-N'-methoxyurea may be crystals, and both single crystal form and crystal mixture are encompassed in the compound of the present invention. Crystals can be produced by crystallization according to a crystallization method known *per se.*

N-(4-(1-(2,6-difluorobenzyl)-5-((dimethylamino)methyl)-3-(6-methoxy-3-pyridazinyl)-2,4-dioxo-1,2,3,4-tetrahydrothieno[2,3-d]pyrimidin-6-yl)phenyl)-N'-methoxyurea may be a hydrate or non-hydrate. Examples of the hydrates include 1 hydrate, 1.5 hydrate and 2 hydrate. When the compound of the present invention is obtained as a mixture of optically active forms, they can be resolved into the desired (R) form or (S) form according to an optical resolution method known *per se.* Moreover, the compound of the present invention may be labeled with an isotope (e.g., ³H, ¹⁴C, ³⁵S).

The nonpeptidic compound having a GnRH antagonistic action is low toxic and shows a superior GnRH antagonistic action and low toxicity. Moreover, it is superior in oral absorbability and action sustainability, as well as stability and pharmacokinetics. Furthermore, its production is easy and simple.

The "in vitro fertilization (IVF)" refers to a method comprising collecting an ovum, fertilizing the ovum in vitro with a spermatozoon and, when cleavage has progressed to a certain degree, inserting the ovum into the uterine cavity. That is, it includes the processes of ovulation induction, ovum collection, in vitro fertilization and culture, and embryo transfer.

The "embryo transfer" refers to, among the in vitro fertilization processes, the process of implanting embryo in the uterine cavity. One to several embryos inserted into the uterine cavity are implanted in the uterus, thereby resulting in pregnancy. The term also encompasses frozen embryo transfer and gamete intrafallopian transfer that do not involve in vitro fertilization.

The "in embryo transfer process" refers to the entire period during which insertion of an embryo or gamete into the uterine cavity or fallopian tube, a sequence of processes of implantation of the embryo or gamete in the uterus and pregnancy, drug administration before and after embryo transfer to achieve pregnancy are performed. The exclusion of "industrial or commercial human embryo transfer processes" is required by Article 53(a) and Rule 28(c) EPC.

The "inhibition of premature ovulation" means inhibiting an ovulation from being ovulated earlier than the timing of ovum collection for in vitro fertilization, due to the ovulation induced by the natural period LH surge. Once natural ovulation occurs, exogenous collection of ovum becomes difficult, and in vitro fertilization cannot be performed.

The compound of the present invention suppresses secretion of gonadotropic hormone in mammals (e.g., human, monkey, bovine, horse, dog, cat, rabbit, rat and mouse) by its GnRH receptor antagonistic action, and can be safely used for promoting and (or) assisting in vitro fertilization (IVF).

The compound of the present invention is used, for example, to collect an ovum in in vitro fertilization. For collecting a good ovum, an ovum is collected by exogenous control rather than natural ovulation, for which the compound of the present invention is used to eliminate an influence of endogenous LH. Specifically, while promoting ovum maturation with an ovulation inducing agent, premature ovulation is inhibited by simultaneous administration of the compound of the present invention. In addition, the compound of the present invention is used in embryo transfer process in in vitro fertilization, frozen embryo transfer and gamete intrafallopian transfer. By eliminating the influence of endogenous LH, from a stage prior to embryo or gamete implantation up to pregnancy through embryo implantation in the uterus after transfer, the uterus can be controlled to a condition suitable for achieving pregnancy.

Since the compound of the present invention is superior in oral absorbability and permits oral administration, it is superior as a promoter and (or) assistant agent for in vitro fertilization or a premature ovulation inhibitor used under controlled ovarian stimulation, as compared to the conventional peptidic GnRH antagonists administered by subcutaneous injection.

Therefore, the compound of the present invention is useful as a premature ovulation inhibitor for use in in vitro fertilization or embryo transfer process, an inhibitor of ovulation induced by endogenous LH in in vitro fertilization and an inhibitor of premature ovulation under controlled ovarian stimulation. Using the compound of the present invention, not only a mature ovum can be obtained certainly, but also the probability or stability of the fertilization of collected ovum, implantation in the uterus, achievement of pregnancy and maintenance of pregnancy can be expected.

The compound of the present invention can be used in combination in the process up to ovum collection in in vitro fertilization, when an ovulation inducing agent such as follicle-stimulating hormone and luteinizing hormone is used to prompt ovum maturation. In addition, the compound of the present invention can be used in embryo transfer process in combination with a promoter for implantation or pregnancy.

The compound of the present invention can be used in combination with gonadotropin (FSH, LH) or a pharmaceutical agent having a gonadotropin-like action; a GnRH superactive agonist such as Leuprorelin acetate, Gonadrelin, Buserelin, Triptorelin, Goserelin, Nafarelin, Histrelin, Deslorelin, Meterelin and lecirelin (preferably Leuprorelin acetate); or a GnRH antagonist such as Cetrorelix, Ganirelix, Abarelix, Nal-Blu, Antide, AzalineB, Degarelix, D63153 and Teverelix.

In addition, the compound of the present invention can also be used in combination with at least one kind of a steroidal or non-steroidal antiandrogen or antiestrogen, a chemotherapeutic agent, a peptidic GnRH antagonist, an α-reductase inhibitor, an α-receptor inhibitor, an aromatase inhibitor, a 17β-hydroxysteroid dehydrogenase inhibitor, an adrenal androgen production inhibitor, a kinase inhibitor, a hormonal therapeutic agent, growth factors or a pharmaceutical agent inhibiting the action of a receptor thereof (hereinafter these are to be also referred to as concomitant drugs).

Examples of the "chemotherapeutic agent" include Ifosfamide, UTF, Adriamycin, Peplomycin, Cisplatin, Cyclophosphamide, 5-FU, UFT, Methotrexate, Mitomycin C and Mitoxantrone.

Examples of the "adrenal androgen production inhibitor" include a lyase (C_{17,20}-lyase) inhibitor.

Examples of the "kinase inhibitor" include tyrosine kinase.

Examples of the "hormonal therapeutic agent" include antiestrogen, progestogen (e.g., MPA and the like), androgen, estrogen and anti-androgen.

The "growth factors" may be any substances as long as it enhances cell growth, and generally, a factor which is a peptide having a molecular weight of not more than 20,000 and exhibits an action at a low concentration on binding with a receptor. Specific examples include (1) EGF (epidermal growth factor) or substances having an activity substantially identical to the activity thereof (e.g., EGF, heregulin (HER2 ligand)), (2) insulin or substances having an activity substantially identical to the activity thereof (e.g., insulin, IGF (insulin-like growth factor)-1, IGF-2), (3) FGF (fibroblast growth factor) or substances having an activity substantially identical to the activity thereof (e.g., aFGF, bFGF, KGF (Keratindcyte Growth Factor), HGF (Hepatocyte Growth Factor), FGF-10 and the like), (4) growth factors (e.g., CSF (colony stimulating factor), EPO (erythropoietin), IL-2 (interleukin-2), NGF (nerve growth factor), PDGF (platelet-derived growth factor), TGFβ (transforming growth factorβ)). The "receptor of the growth factors" may be any receptor capable of binding with the above-mentioned growth factors. Specific examples thereof include EGF receptor, heregulin receptor (HER2), insulin receptor-1, insulin receptor-2, IGF receptor, FGF receptor-1, FGF receptor-2. Examples of the pharmaceutical agent that inhibits the action of the above-mentioned growth factors include herceptin (HER2 receptor antibody). Examples of the pharmaceutical agent that inhibits the action of the above-mentioned growth factors or a receptor thereof include herbimycin, PD153035 (Science 265 (5175) p 1093, (1994)).

Moreover, HER2 inhibitors are also examples of the pharmaceutical agent that inhibits the action of the growth factors or a receptor thereof. The HER2 inhibitor may be any of antibodies, low-molecular-weight compounds (synthetic compounds, naturally occurring substances), antisenses, HER2 ligands, heregulin and those having a partially modified or altered structure, as long as it inhibits the activity of HER2 (e.g., phosphorylation activity). Furthermore, it may be a substance that inhibits the HER2 activity by inhibiting HER2 receptor (e.g., HER2 receptor antibody). Examples of the low-molecular-weight compound that has an HER2 inhibitory activity include compounds described in WO98/03505, specifically 1-[3-[4-[2-((E)-2-phenylethenyl)-4-oxazolylmethoxy]phenyl]propyl]-1,2,4-triazole.

For breast cancer patients, the compound of the present invention may be used in combination with a pharmaceutical agent such as GnRH superactive agonist, antiestrogen, chemotherapeutic agent [e.g., Cyclophosphamide, 5-FU, UFT, Methotrexate, Adriamycin, Mitomycin C, Mitoxantrone and the like], peptidic GnRH antagonist, aromatase inhibitor, adrenal androgen production inhibitor, kinase inhibitor, hormonal therapeutic agent [e.g., antiestrogen (e.g., Tamoxifen), progestogen (e.g., MPA), androgen and estrogen] and a pharmaceutical agent that inhibits the action of growth factors or receptors thereof.

By combining the compound of the present invention and a concomitant drug, a superior effect such as
(1) the dose can be reduced as compared to single administration of the compound of the present invention or a concomitant drug,
(2) the drug to be used in combination with the compound of the present invention can be selected according to the condition of patients (mild case, severe case and the like),
(3) the period for inhibiting premature ovulation can be set longer by selecting a concomitant drug having different action and mechanism from those of the compound of the present invention,
(4) a sustained effect of premature ovulation inhibition can be designed by selecting a concomitant drug having different action and mechanism from those of the compound of the present invention,
(5) a synergistic effect can be afforded by a combined use of the compound of the present invention and a concomitant drug,
(6) side effects can be reduced by the combined use of the compound of the present invention and a concomitant drug,
(7) the compound of the present invention can also be administered to breast cancer patients and the like by the combined use of a concomitant drug, can be achieved.

In the following, a combined use of the compound of the present invention with a concomitant drug is referred to as "the combination agent of the present invention".

When the compound of the present invention and the aforementioned concomitant drug is used in combination, the dose can be appropriately determined with the recommended minimum clinical dose of individual drugs as the standard, and in consideration of the age and body weight of the administration subject, symptom, administration time, administration method, dosage form and combination of drugs. The dose of a particular patient is determined according to the age, body weight, general health status, sex, diet, administration time, administration method, clearance rate, drug combination, severity of the disease for which the patient is undergoing treatments, and other factors.

Typically, the respective daily dose of the compound of the present invention and at least one compound selected from various concomitant drugs or a salt thereof when used in combination is from not less than about 1/50 of the minimum recommended clinical dose to not more than the maximum recommended level of actual single administration thereof.

When using the combination agent of the present invention, the administration time of the compound of the present invention and the concomitant drug is not restricted, and the compound of the present invention or a pharmaceutical composition thereof and the concomitant drug or a pharmaceutical composition thereof can be administered to an administration subject simultaneously, or may be administered in a staggered manner. The dosage of the concomitant drug may be determined according to the administration amount clinically used, and can be appropriately selected depending on an administration subject, administration route, disease and combination.

The administration mode of the concomitant drug is not particularly limited, and the compound of the present invention and the concomitant drug only need to be combined on administration. Examples of such administration mode include the following:
(1) administration of a single preparation obtained by simultaneously formulating the compound of the present invention and the concomitant drug, (2) simultaneous administration of two kinds of preparations of the compound of the present invention and the concomitant drug, which have been separately formulated, by the same administration route, (3) administration of two kinds of preparations of the compound of the present invention and the concomitant drug, which have been separately formulated, by the same administration route in a staggered manner, (4) simultaneous administration of two kinds of preparations of the compound of the present invention and the concomitant drug, which have been separately formulated, by different administration routes, (5) administration of two kinds of preparations of the compound of the present invention and the concomitant drug, which have been separately formulated, by different administration routes in a staggered manner (for example, administration in the order of the compound of the present invention and the concomitant drug, or in the reverse order).

When the compound of the present invention is used as the above-mentioned promoter or pharmaceutical aid (a premature ovulation inhibitor for use in in vitro fertilization or embryo transfer process), both oral administration and parenteral administration are available according to a method known *per se.* The compound is mixed with a pharmaceutically acceptable carrier, and generally administered orally in the solid dosage form of tablet, capsule, granule and powder. Alternatively, it is parenterally administered intravenously, subcutaneously and intramuscularly in the form of injection, suppository and sublingual tablet In addition, the compound may be administered sublingually, subcutaneously and intramuscularly as a sustained release preparation such as sublingual tablet and microcapsule.

While the daily dose of the compound of the present invention varies depending on the level of symptoms; age, sex, body weight and sensitivity difference of the subject of administration; timing of administration, interval, roperties, dispensing and kind of pharmaceutical preparation and kind of the active ingredient, and is not particularly limited, when used for the inhibition of premature ovulation in in vitro fertilization, the compound is generally administered in a dose of about 0.01 - 30 mg, preferably about 0.02 - 10 mg, more preferably 0.1 - 10 mg, most preferably 0.1 - 5 mg, per 1 kg body weight of a mammal, generally in 1 - 4 portions a day. When used for the inhibition of premature ovulation in embryo transfer process, the compound is generally administered in a dose of about 0.01 - 30 mg, preferably about 0.02 - 10 mg, more preferably 0.1 - 10 mg, most preferably 0.1 - 5 mg, per 1 kg body weight of a mammal, generally in 1 - 4 portions a day. While the dose for use in the animal husbandry or marine industry field is in accordance with the above-mentioned doses, the compound is generally administered in a dose of about 0.01 - 30 mg, preferably about 0.1 - 10 mg, per 1 kg body weight of an administration subject organism, generally in 1 - 3 portions a day. The content of compound (I) in the pharmaceutical composition of the present invention is about 0.01 to 100 wt% of the whole composition.

As the above-mentioned pharmaceutically acceptable carrier, various organic or inorganic carrier substances conventionally used as preparation materials can be used, and admixed as excipient, lubricant, binder and disintegrant for solid preparations; or solvent, solubilizing agent, suspending agent, isotonicity agent, buffer and shooting agent for liquid preparations. Where necessary, preparation additives such as preservative, antioxidant, coloring agent and sweetening agent can be used. Preferable examples of the above-mentioned excipient include lactose, sucrose, D-mannitol, starch, crystalline cellulose and light anhydrous silicic acid. Preferable examples of the above-mentioned lubricant include magnesium stearate, calcium stearate and talc, colloidal silica. Preferable examples of the above-mentioned binder include crystalline cellulose, sucrose, D-mannitol, dextrin, hydroxypropylcellulose, hydroxypropylmethylcellulose and polyvinylpyrrolidone. Preferable examples of the above-mentioned disintegrant include starch, carboxymethylcellulose, calcium carboxymethylcellulose, croscarmellose sodium and carboxymethyl starch sodium. Preferable examples of the above-mentioned solvent include water for injection, alcohol, propylene glycol, macrogol, sesame oil and corn oil. Preferable examples of the above-mentioned solubilizing agents include polyethylene glycol, propylene glycol, D-mannitol, benzyl benzoate, ethanol, trisaminomethane, cholesterol, triethanolamine, sodium carbonate and sodium citrate. Preferable examples of the above-mentioned suspending agent include surfactants such as stearyltriethanolamine, sodium lauryl sulfate, lauryl aminopropionic acid, lecithin, benzalkonium chloride, benzethonium chloride and glycerol monostearate; and hydrophilic polymers such as polyvinyl alcohol, polyvinylpyrrolidone, sodium carboxymethylcellulose, methylcellulose, hydroxymethylcellulose, hydroxyethylcellulose and hydroxypropylcellulose. Preferable examples of the above-mentioned isotonicity agent include sodium chloride, glycerol and D-mannitol. Preferable examples of the above-mentioned buffer include phosphate buffer, acetate buffer, carbonate buffer and citrate buffer. Preferable examples of the soothing agent include benzyl alcohol. Preferable examples of the above-mentioned preservative include paraoxybenzoates, chlorobutanol, benzyl alcohol, phenethyl alcohol, dehydroacetic acid and sorbic acid. Preferable examples of the above-mentioned antioxidant include sulfite and ascorbic acid.

The compound of the present invention can be formed into an intravenous, subcutaneous or intramuscular injection by adding a suspending agent, a solubilizing agent, a stabilizer, a tonicity agent and a preservative and according to a method known per *se.* In this case, a freeze-dried product may be produced as necessary according to a method known per se. When the compound of the present invention is administered to, for example, a human, the compound can be safely administered orally or parenterally as it is or in the form of a pharmaceutical composition produced by admixing with an appropriate pharmacologically acceptable carrier, excipient or diluent. Examples of the above-mentioned pharmaceutical composition include oral preparations (e.g., powder, granule, capsule, tablet), and parenteral agents [e.g., injection, drip infusion, external preparation (e.g., preparation for nasal administration and dermal preparation), suppositories (e.g., rectal suppository and vaginal suppository )]. These preparations can be produced according to a method known *per se* generally used for preparation forming steps.

The compound of the present invention can be formulated into an injection such as an aqueous injection in combination with a dispersing agent (e.g., Tween 80 (manufactured by Atlas Powder, US), HCO60 (manufactured by Nikko Chemicals) polyethylene glycol, carboxymethylcellulose and sodium alginate), a preservative (e.g., methylparaben, propylparaben and benzyl alcohol) and an isotonycity agent (e.g., sodium chloride, mannitol, sorbitol and glucose ), or an oily injection by dissolving, suspending or emulsifying in a vegetable oil such as olive oil, sesame oil, cottonseed oil and corn oil, or propylene glycol. To formulate an oral preparation, the compound of the present invention is mixed with, for example, an excipient (e.g., lactose, sucrose starch), a disintegrant (e.g., starch, calcium carbonate), a binder (e.g., starch, gum arabic, carboxymethylcellulose, polyvinylpyrrolidone, hydroxypropylcellulose), a lubricant (e.g., talc, magnesium stearate, polyethylene glycol 6000) and subjected to compression molding according to a method known *per se,* and where necessary, coating for taste masking, and enteric coating or sustainability according to a method known *per se.* Examples of the coating agent to be used therefor include hydroxypropylmethylcellulose, ethylcellulose, hydroxymethylcellulose, hydroxypropylcellulose, polyoxyethyleneglycol, Tween 80, pluronic F₆₈, cellulose acetate phthalate, hydroxypropylmethylcellulose phthalate, hydroxymethylcellulose acetate succinate, Eudragit (manufactured by Rohm, Germany, methacrylic acid/acrylic acid copolymer) and dye (e.g., red iron oxide, titanium dioxide, etc.). When formulating an enteric preparation, an intermediate phase may be formed according to a method known *per se* for the purpose of separating an enteric phase and a drug-containing phase.

To formulate an external preparation, the compound of the present invention may be formulated into a solid, semisolid or liquid external preparation according to a method known *per se.* For example, to provide the above-mentioned solid preparation, the compound of the present invention may be directly used, or formulated into a powder composition by adding to mix with an excipient (e.g., glycol, mannitol, starch, microcrystalline cellulose and the like), a thickener (e.g., natural gum, cellulose derivative and acrylic acid polymer). To provide the above-mentioned liquid preparation, an oily or aqueous suspension is formulated mostly in the same manner as in the injection. In the case of a semisolid preparation, an aqueous or oily gel, or an ointment form is preferable. They may contain a pH adjusting agent (e.g., carbonic acid, phosphoric acid, citric acid, hydrochloric acid and sodium hydroxide) and a preservative (e.g., paraoxybenzoate; chlorobutanol, benzalkonium chloride ). To provide a suppository, for example, the compound of the present invention can be formulated into an oily or aqueous solid, semisolid or liquid suppository according to a method known *per se.* Examples of the oily base to be used for the above-mentioned compositions include higher fatty acid glycerides [e.g., cacao butter, witepsol (manufactured by Dynamitnovel Ltd., Germany)], intermediate grade fatty acids [e.g., miglyol (manufactured by Dynamitnovel Ltd., Germany)] and vegetable oil (e.g., sesame oil, soybean oil, cottonseed oil). Moreover, examples of the aqueous base include polyethylene glycol and propylene glycol, and examples of the aqueous gel base include natural gums, cellulose derivatives, vinyl polymers and acrylic acid polymers.

The present invention is explained in more detail in the following by referring to Formulation Examples, Reference Examples and Examples.

¹H-NMR spectra are measured with tetramethylsilane as the internal standard, using VARIAN GEMINI 200 (200 MHz type spectrometer), JEOL Ltd. (JEOL) LAMBDA300 (300 MHz type spectrometer) or BRUKER AM 500 (500 MHz type spectrometer); all δ values are expressed in ppm. Unless otherwise specified, "%" shows weight percent. However, the yield is in mol/mol%. Other symbols in the present specification mean the following.
s: singlet
d: doublet
t: triplet
dt: double triplet
m: multiplet
br: broad

The room temperature means, but is not particularly strictly limited to, the range of from about 15°C to 25°C. In addition, lactose, cornstarch and magnesium stearate used in the Formulation Examples and Examples were the Japanese Pharmacopoeia 14th Edition or Japanese Pharmaceutical Excipients 2003 compatible products.

### Examples

### Formulation Example 1

| | |
|---|---|
| (1) compound A | 1 g |
| (2) lactose | 197 g |
| (3) corn starch | 50 g |
| (4) magnesium stearate | 2 g |

The above-mentioned (1) and (2), and corn starch (20 g) were admixed, and granulated together with a paste produced from corn starch (15 g) and water (25 mL). Corn starch (15 g) and the above-mentioned (4) was added thereto, and the mixture was compressed by a tablet compression machine to give tablets (2000 tablets, diameter of 3 mm) containing 0.5 mg of compound A per tablet.

### Formulation Example 2

| | |
|---|---|
| (1) compound A | 2 g |
| (2) lactose | 197 g |
| (3) corn starch | 50 g |
| (4) magnesium stearate | 2 g |

In the same manner as in Formulation Example 1, tablets (2000 tablets, diameter 3 mm) containing 1.0 mg of compound A per tablet were produced.

### Formulation Example 3

| | |
|---|---|
| (1) compound A | 5.0 mg |
| (2) lactose | 60.0 mg |
| (3) corn starch | 35.0 mg |
| (4) gelatin | 3.0 mg |
| (5) magnesium stearate | 2.0 mg |

A mixture of the above-mentioned (1), (2) and (3) was granulated by passing through a 1 mm mesh sieve while using 10% aqueous gelatin solution (0.03 ml, 3.0 mg as gelatin). The granules were dried at 40°C, and sieved again. The obtained granules were mixed with the above-mentioned (5), and compressed. The obtained core tablet was coated with an aqueous sugar coating suspension of saccharose, titanium dioxide, talc and gum arabic. The coated tablet was glazed with beeswax to give a coated tablet.

### Reference Example 1

### 2-amino-4-methyl-5-(4-nitrophenyl)thiophene-3-carboxylic acid ethyl ester

A mixture of 4-nitrophenylacetone (35.0 g, 195 mmol), ethyl cyanoacetate (23.8 g, 195 mmol), ammonium acetate (3.1 g, 40 mmol) and acetic acid (9.1 ml, 159 mmol) was heated under reflux for 24 hr while removing the resulting water by a Dean-Stark trap. After cooling, the reaction mixture was concentrated under reduced pressure, and the residue was partitioned between dichloromethane and aqueous sodium hydrogen carbonate solution. The organic layer was washed with brine and dried (MgSO₄), after which the solvent was evaporated under reduced pressure. The residue was purified by silica gel column chromatography. The obtained oily substance was dissolved in ethanol, sulfur (5.0 g, 160 mmol) and diethylamine (16.0 ml, 160 mmol) were added, and the mixture was stirred at 60-70°C for 2 hr. After cooling, the reaction mixture was concentrated under reduced pressure, and the residue was partitioned between dichloromethane and aqueous sodium hydrogen carbonate solution. The organic layer was washed with brine and dried (MgSO₄), after which the solvent was evaporated under reduced pressure. The residue was purified by silica gel column chromatography, and crystallized from ether-hexane to give the title compound (22.2 g, 52%) as red plate crystals.
mp: 168-170°C (recrystallization from ether-hexane). elemental analysis value for C₁₄H₁₄N₂O₄S

| | C(%) | H(%) | N(%) |
|---|---|---|---|
| Calculated: | 54.89; | 4.61; | 9.14 |
| Found: | 54.83; | 4.90; | 9.09 |

¹H-NMR (200 MHz, CDCl₃) δ: 1.39 (3H, t, J = 7.1 Hz), 2.40 (3H, s), 4.34 (2H, q, J = 7.1 Hz), 6.27 (2H, br), 7.48 (2H, d, J = 8.7 Hz), 8.23 (2H, d, J = 8.7 Hz).
IR (KBr): 3446, 3324, 1667, 1580, 1545, 1506, 1491, 1475, 1410, 1332 cm⁻¹.

### Reference Example 2

### 5-methyl-6-(4-nitrophenyl)-3-phenylthieno[2,3-d]pyrimidine-2,4(1H,3H)-dione

To a solution of the compound (5.00 g, 16.32 mmol) obtained in Reference Example 1 in pyridine (30 ml) was added phenylisocyanate (2.66 ml, 24.48 mmol), and the mixture was stirred at 45°C for 6 hr. The reaction mixture was concentrated under reduced pressure and the obtained residue was treated to give an ethanol (6 ml) solution. 28% Sodium methoxide (7.86 g, 40.80 mmol) was added to the solution, and the reaction mixture was stirred at room temperature for 2 hr. 2N Hydrochloric acid (25 ml, 50 mmol) was added, and the ethanol solvent was evaporated under reduced pressure. The obtained residue was filtrated and washed with water-ethanol. After drying under reduced pressure, the residue was recrystallized from ethanol to give the title compound (6.09 g, 98%) as a yellow powder.
mp: >300°C.
elemental analysis value for C₁₉H₁₃N₃O₄S·0.3H₂O

| | C(%) | H(%) | N(%) |
|---|---|---|---|
| Calculated: | 59.30; | 3.56; | 10.92 |
| Found: | 59.56; | 3.52; | 10.93 |

¹H-NMR (300MHz, DMSO-d₆) δ: 2.50 (3H, s), 7.31-7.46 (5H, m), 7.78 (2H, d, J = 8.8 Hz), 8.32 (2H, d, J = 8.8 Hz), 12.50 (1H, s).
IR (KBr): 1715, 1657, 1593, 1510 cm⁻¹.

### Reference Example 3

### 1-(2,6-difluorobenzyl)-5-methyl-6-(4-nitrophenyl)-3-phenylthieno[2,3-d]pyrimidine-2,4(1H,3H)-dione

To a solution of the compound (52.54 g, 0.131 mol) obtained in Reference Example 2 in dimethylformamide (1.0 1) were added potassium carbonate (19.00 g, 0.138 mol), potassium iodide (22.90 g, 0.138 mol) and 2,6-difluorobenzylchloride (22.40 g, 0.138 mol), and the mixture was stirred at room temperature for 2 hr. The reaction mixture was concentrated, and the obtained residue was partitioned between chloroform and brine. The aqueous layer was extracted with chloroform. The extracts were combined and washed with brine and dried (MgSO₄), after which the solvent was evaporated under reduced pressure. The obtained residue was purified by silica gel column chromatography to give the title compound (61.50 g, 93%) as pale-yellow crystals.
mp: 280-282°C.
elemental analysis value for C₂₆H₁₇N₃O₄SF₂

| | C(%) | H(%) | N(%) |
|---|---|---|---|
| Calculated: | 61.78; | 3.39; | 8.31 |
| Found: | 61.67; | 3.46; | 8.21 |

¹H-NMR (300 MHz, CDCl₃) δ: 2.57 (3H, s), 5.38 (2H, s), 6.94 (2H, d, J = 8.1 Hz), 7.42-7.58 (8H, m), 8.29 (2H, d, J = 8.8 Hz).
IR (KBr): 1719, 1669, 1524, 1473 cm⁻1.

### Reference Example 4

### 5-bromomethyl-1-(2,6-difluorobenzyl)-6-(4-nitrophenyl)-3-phenylthieno[2,3-d]pyrimidine-2,4(1H,3H)-dione

A mixture of the compound (30.34 g, 0.060 mol) obtained in Reference Example 3, N-bromosuccinimide (12.81 g, 0.072 mol), α,α'-azobisisobutyronitrile (1.15 g, 0.007 mol) and chlorobenzene (450 ml) was stirred at 85°C for 3 hr. After cooling, the reaction mixture was washed with brine and dried (MgSO₄), after which the solvent was evaporated under reduced pressure. The obtained residue was recrystallized from ethyl acetate to give the title compound (80.21 g, 100%) as yellow needle-like crystals.
mp: 228-229°C.
¹H-NMR (300 MHz, CDCl₃) δ: 4.77 (2H, s), 5.38 (2H, s), 6.96 (2H, t, J = 8.1 Hz), 7.29-7.58 (6H, m), 7.79 (2H, d, J = 8.5 Hz), 8.35 (2H, d, J = 8.5 Hz).
IR (KBr): 1721, 1680, 1524, 1473, 1348 cm⁻¹.
FAB-Mass m/z 584 (MH)⁺

### Reference Example 5

### 5-(N-benzyl-N-methylaminomethyl)-1-(2,6-difluorobenzyl)-6-(4-nitrophenyl)-3-phenylthieno[2,3-d]pyrimidine-2,4(1H,3H)-dione

To a solution of the compound (80.00 g, 0.119 mol) obtained in Reference Example 4 in dimethylformamide (600 ml) were added ethyldiisopropylamine (27.00 ml, 0.155 mol) and benzylmethylamine (18.45 ml, 0.143 mol) under ice-cooling. The mixture was stirred at room temperature for 2 hr. The reaction mixture was concentrated, and the obtained residue was partitioned between ethyl acetate and saturated aqueous sodium hydrogen carbonate solution. The aqueous layer was extracted with ethyl acetate, and the organic layers were combined and dried (mugSO₄), after which the solvent was evaporated under reduced pressure. The obtained residue was purified by silica gel column chromatography to give a yellow oil (74.90 g, 100%), which was recrystallized from ethyl acetate to give the title compound as yellow needle-like crystals.
mp: 173-174°C.
elemental analysis value for C₃qH₂₆N₄O₄SF₂·0.5H₂O

| | C(%) | H(%) | N(%) |
|---|---|---|---|
| Calculated: | 64.45; | 4.29; | 8.84 |
| Found: | 64.50; | 4.24; | 8.82 |

¹H-NMR (300 MHz, CDCl₃) [free amine] 8: 1.31 (3H, s), 3.60 (2H, s), 3.96 (2H, s), 5.39 (2H,s), 6.95 (2H, t, J = 8.2 Hz), 7.18-7.55 (11H, m), 8.02 (2H, d, J = 9.0 Hz), 8.26 (2H, d, J = 9.0 Hz).
IR (KBr) [hydrochloride]: 1719, 1678, 1597, 1520 cm⁻¹.

### Reference Example 6

### 6-(4-aminophenyl)-5-(N-benzyl-N-methylaminomethyl)-1-(2,6-difluorobenzyl)-3-phenylthieno[2,3-d]pyrimidine-2,4(1H,3H)-dione

To a solution of the compound (3.00 g, 4.80 mmol) obtained in Reference Example 5 in formic acid (30 ml) were added 1M hydrogen chloride-ether (14.4 ml, 14.4 mmol) and 10% palladium carbon powder (300 mg) under ice-cooling. The mixture was stirred for 2 hr at ambient temperature under normal pressure, and hydrogenated. The reaction mixture was filtered through celite. The filtrate was concentrated under reduced pressure, and the obtained residue was partitioned between dichloromethane and saturated aqueous sodium hydrogen carbonate solution. The aqueous layer was extracted with dichloromethane, the organic layers were combined and dried (MgSO₄), after which the solvent was evaporated under reduced pressure. The obtained residue was purified by silica gel column chromatography to give the title compound (2.41 g, 84%) as white crystals.
mp: 205-207°C.
elemental analysis value for C₃₄H₂₈N₄O2SF2·0.1AcOEt·1.2H₂O

| | C(%) | H(%) | N(%) |
|---|---|---|---|
| Calculated: | 66.09; | 5.03; | 8.96 |
| Found: | 66.93; | 4.94; | 8.67 |

¹H-NMR (300 MHz, CDCl₃) δ: 2.05 (3H, s), 3.56 (2H, s), 3.83 (2H, br), 3.88 (2H, s), 5.36 (2H, s), 6.70 (2H, d, J = 8.8 Hz), 6.88-6.94 (2H, m), 7.21-7.31 (8H, m), 7.41-7.53 (5H, m).
IR (KBr): 1715, 1657, 1628, 1537 cm⁻¹.

### Reference Example 7

### 5-(N-benzyl-N-methylaminomethyl)-1-(2,6-difluorobenzyl)-6-[4-(3-methoxyureido)phenyl]-3-phenylthieno[2,3-d]pyrimidine-2,4(1H,3H)-dione (compound A)

To a solution of the compound (5.0 g, 8.41 mmol) obtained in Reference Example 6 in dichloromethane (120 ml) was added triethylamine (2.34 ml, 16.82 mmol) under ice-cooling, and the mixture was stirred. Under ice-cooling, N,N'-carbonyldiimidazole (2.73 g, 16.82 mmol) was added to the reaction mixture. The temperature of the mixture was raised from ice-cooling to room temperature and stirred for 42 hr. The mixture was placed under ice-cooling again, and O-methylhydroxylamine hydrochloride (7.02 g, 84.08 mmol) and triethylamine (11.7 ml, 84.08 mmol) were added. The reaction mixture was raised from ice-cooling to room temperature and stirred for 3 hr. The reaction mixture was partitioned between chloroform and saturated aqueous sodium hydrogen carbonate solution. The aqueous layer was extracted with chloroform. The extracts were combined and washed with brine and dried (MgSO₄), after which the solvent was evaporated under reduced pressure. The obtained residue was purified by silica gel column chromatography to give a pale-yellow solid, which was recrystallized from chloroform-ether to give the title compound (4.52 g, 80%) as white crystals.
mp:204-205°C.
elemental analysis value for C₃₆H₃₁NsO₄SF₂

| | C(%) | H(%) | N(%) |
|---|---|---|---|
| Calculated: | 64.75; | 4.68; | 10.49 |
| Found: | 64.61; | 4.67; | 10.31 |

¹H-NMR (300 MHz, CDCl₃) δ: 2.05 (3H, s), 3.57 (2H, s), 3.82 (3H, s), 3.90 (2H, s), 5.37 (2H, s), 6.92 (2H, d, J = 8.2 Hz), 7.16-7.31 (9H, m), 7.42-7.57 (5H, m), 7.63 (1H, s), 7.73 (2H, d, J = 8.8 Hz).
IR (KBr): 3338, 3064, 1717, 1669, 1628, 1591, 1531, 1470 cm⁻¹. Reference Example 8

### 5-(N-benzyl-N-methylaminomethyl)-1-(2,6-difluorobenzyl)-6-[4-(3-methoxyureido)phenyl]-3-phenylthieno[2,3-d]pyrimidine-2,4(1H,3H)-dione hydrochloride

To a solution of the white crystals (38.34 g, 57.42 mmol) obtained in Reference Example 7 in dichloromethane (800 ml) was added 1M hydrogen chloride in ether (100 ml) under ice-cooling, and the mixture was stirred at the same temperature for 10 min. The reaction mixture was concentrated under reduced pressure, and the obtained residue was recrystallized from methanol-ether to give the title compound (40.0 g, 99%) as white powder crystals.
mp: 182-185°C.
elemental analysis value for C₃₆H₃₁N₅O₄SF₂·HCl·0.5H₂O

| | C(%) | H(%) | N(%) |
|---|---|---|---|
| Calculated: | 60.63; | 4.66; | 9.82 |
| Found: | 60.45; | 4.68; | 9.62 |

IR(KBr): 3440, 3042, 1713, 1665, 1628, 1593, 1539, 1473 cm⁻¹. FAB-Mass m/z 668 (MH)⁺

### Example 1

Using the compound (100 mg) produced in Reference Example 7, lactose (165 mg), corn starch (25 mg), polyvinyl alcohol (4 mg) and magnesium stearate (1 mg), a tablet is produced according to a conventional method.

### Example 2

Using the compound (100 mg) produced in Reference Example 8, lactose (165 mg), corn starch (25 mg), polyvinyl alcohol (4 mg) and magnesium stearate (1 mg), a tablet is produced according to a conventional method.

### Example 3

Using the compound (1 g) produced in Reference Example 7, lactose (197 g), corn starch (50 g) and magnesium stearate (2 g), a tablet is produced according to a conventional method.

### Example 4

Using the compound (1 g) produced in Reference Example 8, lactose (197 g), corn starch (50 g) and magnesium stearate (2 g), a tablet is produced according to a conventional method.

### Reference Example 9

### Production of 2-[N-(2,6-difluorobenzyl)-N-ethoxycarbonyl]amino-4-[N-(2-methoxyethyl)-N-methylaminomethyl]-5-(4-aminophenyl)thiophene-3-carboxylic acid ethyl ester

To a solution of 2-[N-(2,6-difluorobenzyl)-N-ethoxycarbonyl]amino-4-[N-(2-methoxyethyl)-N-methylaminomethyl]-5-(4-nitrophenyl)thiophene-3-carboxylic acid ethyl ester (12.43 g) (JP-A-2001-278884, WO00/56739) in ethanol (315 ml) were added 2N-hydrogen chloride/diethyl ether solution (21 ml) and 10% palladium/carbon containing 50% water (3.73 g), and the mixture was vigorously stirred for 1 hr under a hydrogen atmosphere. The filtrate free of the catalyst was neutralized with aqueous sodium hydrogen carbonate solution, after which the solvent was evaporated. The obtained residue was partitioned between ethyl acetate/water. The organic layer was washed with saturated brine, and dried over anhydrous magnesium sulfate. The solvent was evaporated under reduced pressure, and the obtained residue was purified by NH-silica gel (manufactured by Fuji Silysia Chemical Ltd.) chromatography to give the title compound (11.44 g) as an oil.
¹H-NMR (CDCl₃) δ: 1.12-1.30 (3H, br), 2.05 (3H, s), 2.39 (2H, t, J = 6.3 Hz), 3.27 (3H, s), 3.32 (3H, t, J = 6.3 Hz), 3.59 (2H, s), 3.78 (2H, s), 4.20 (2H, q, J = 7.1 Hz), 4.10-4.23 (2H, br), 5.00 (2H, s), 6.66 (2H, d, J = 8.6 Hz), 6.84 (2H, t, J = 8.2 Hz), 7.18 (2H, d, J = 8.6 Hz), 7.15-7.30 (1H, m).
IR (KBr): 1717, 1626, 1609, 1472, 1406, 1300, 1246 cm⁻¹.

### Reference Example 10

### Production of 2-[(2,6-difluorobenzyl)(ethoxycarbonyl)amino]-5-(4-{[(methoxyamino)carbonyl]amino}phenyl)-4-{[(2-methoxyethyl)(methyl)amino]methyl}-3-thiophenecarboxylic acid ethyl ester

To a solution (113 ml) of the compound (4.89 g) of Reference Example 9 in dichloromethane was added N-ethyldiisopropylamine (3.06 ml) under ice-cooling, and the mixture was stirred. N,N'-Carbonyldiimidazole (2.82 g) was added to the reaction mixture under ice-cooling. The temperature of the mixture was raised from ice-cooling to room temperature, and the mixture was stirred for 67 hr. The mixture was placed under ice-cooling again, and O-methylhydroxyamine hydrochloride (7.26 g) and N-ethyldiisopropylamine (15.6 ml) were added. The temperature of the reaction mixture was raised from ice-cooling to room temperature, and the mixture was stirred for 19 hr. The mixture was partitioned between chloroform and saturated aqueous sodium hydrogen carbonate solution. The aqueous layer was extracted with chloroform, and the extracts were combined and washed with brine and dried (MgSO₄), after which the solvent was evaporated under reduced pressure. The obtained residue was purified by silica gel column chromatography to give the title compound (4.89 g) in a pale-yellow caramel form.
¹H-NMR (CDCl₃ δ: 1.19 (3H, brs), 1.30 (3H, t, J = 6.9 Hz), 2.04 (3H, s), 2.40 (2H, t, J = 6.0 Hz), 3.27 (3H, s), 3.33 (2H, t, J = 6.0 Hz), 3.60 (2H, s), 3.81 (3H, s), 4.13-4.24 (4H, m), 5.00 (2H, s), 6.84 (2H, t, J = 7.8 Hz), 7.19-7.29 (2H, m), 7.36 (2H, d, J = 8.7 Hz), 7.50 (2H, d, J = 8.7 Hz), 7.60 (1H, s).
IR (KBr): 1717, 1590, 1528, 1472, 1408, 1304 cm⁻¹.

### Reference Example 11

### Production of 2-[(2,6-difluorobenzyl)(ethoxycarbonyl)amino]-5-(4-{[(methoxyamino)carbonyl]amino}phenyl)-4-{[(2-methoxyethyl)(methyl)amino]methyl}-3-thiophenecarboxylic acid

To a solution of the compound (4.81 g) of Reference Example 10 in ethanol (114 ml) was added 2N sodium hydroxide solution (18.9 ml), and the mixture was stirred at 60°C for 5 hr. The mixture was cooled to room temperature, 1N hydrochloric acid (37.8 ml) was added, and the solvent was evaporated. The obtained residue was dissolved in ethanol and toluene, and the solvent was evaporated again. Anhydrous ethanol (30 ml) was added to the residue, and the inorganic product was filtrated. The filtrate was concentrated to dryness, and the obtained residue was triturated with anhydrous ether, collected by filtration and dried to give the title compound (4.43 g).
¹H-NMR (CDCl₃) δ: 1.17 (3H, brs), 2.45 (3H, s), 2.81 (2H, brs), 3.28 (3H, s), 3.55 (2H, t, J = 4.8 Hz), 3.82 (3H, s), 3.92 (2H, s), 4.10-4.35 (2H, m), 5.06 (2H, s), 6.82 (2H, t, J = 7.8 Hz), 7.16 (2H, d, J = 8.4 Hz), 7.22-7.35 (1H, m), 7.60 (2H, d, J = 8.4 Hz), 8.00-8.50 (2H, br).IR (KBr): 1713, 1605, 1528, 1472, 1408 cm⁻¹.

### Reference Example 12

### Production of N-(4-(1-(2,6-difluorobenzyl)-5-(((2-methoxyethyl)(methyl)amino)methyl)-2,4-dioxo-3-(2-pyridinyl)-1,2,3,4-tetrahydrothieno[2,3-d]pyrimidin-6-yl)phenyl)-N'-methoxyurea

To a solution of the compound (607 mg) obtained in Reference Example 11 and 2-aminopyridine (142 mg) in DMF (10 ml) were added diethyl cyanophosphate (245 mg) and N-ethyldiisopropylamine (284 µl). The mixture was gradually warmed to room temperature and stirred for 13 hr, after which the reaction mixture was partitioned between ethyl acetate/water. The organic layer was washed successively with water and saturated brine and dried over anhydrous sodium sulfate. The residue obtained by evaporating the solvent under reduced pressure was crudely purified by aminopropylsilica gel (manufactured by Fuji Silysia Chemical Ltd.) chromatography. The obtained crude amide form (350 mg) was dissolved in ethanol (25.5 ml), a solution (196 mg) of 28%-sodium methoxide in methanol was added, and the mixture was stirred at room temperature for 15 hr. The mixture was neutralized with 1N-hydrochloric acid (1 ml), the solvent was evaporated, and the residue was partitioned between ethyl acetate/water. The organic layer was washed successively with water and saturated brine and dried over anhydrous sodium sulfate. The residue obtained by evaporating the solvent under reduced pressure was purified by aminopropylsilica gel (manufactured by Fuji Silysia Chemical Ltd.) chromatography (45 g; developer; ethyl acetate/hexane:7/3→ethyl acetate) and recrystallized from THF-ethanol to give the title compound (210 mg) as colorless crystals.
elemental analysis for C₃₁H₃₀N₆O₅SF₂

| | |
|---|---|
| Calculated: | C, 58.48; H, 4.75; N, 13.20. |
| Found: | C, 58.46; H, 4.68; N, 12.93. |

¹H-NMR (CDCl₃) δ: 2.15 (3H, s), 2.62 (2H, t, J = 5. 9 Hz), 3.26 (3H, s), 3.41 (2H, t, J = 5.9 Hz), 3.80 (3H, s), 3.81 (2H, brs), 5.34 (2H, brs), 6.91 (2H, t, J = 8.1 Hz), 7.24-7.40 (4H, m), 7.53 (2H, d, J = 8.4 Hz), 7.62 (2H, d, J = 8.4 Hz), 7.65 (1H, s), 7.88 (1H, dt, J = 1.5 Hz, 7.8 Hz), 8.67-8.69 (1H, m).
IR (KBr): 1717, 1674, 1591, 1530, 1460, 1329 cm⁻¹.

### Reference Example 13

### Production of N-(4-(5-((benzyl(methyl)amino)methyl)-1-(2,6-difluorobenzyl)-2,4-dioxo-3-(2-pyridinyl)-1,2,3,4-tetrahydrothieno[2,3-d]pyrimidin-6-yl)phenyl)-N'-methoxyurea

To a solution (20 ml) of 4-(N-benzyl-N-methylaminomethyl)-2-[N-(2,6-difluorobenzyl)-N-ethoxycarbonyl]amino-5-[4-(3-methoxyureido)phenyl]thiophene-3-carboxylic acid (2.40 g, 3.76 mmol) and 2-aminopyridine (1.06 g, 11.28 mmol) in DMF were added ethyldiisopropylamine (1.05 ml, 6.02 mmol) and diethyl cyanophosphorate (0.86 ml, 5.64 mmol), and the mixture was stirred at room temperature for 3 days. An aqueous sodium hydrogen carbonate solution was added, the mixture was extracted with ethyl acetate, and the organic layer was washed with brine. After drying over magnesium sulfate, the mixture was concentrated under reduced pressure, and the residue was purified by silica gel column chromatography (eluate; ethyl acetate) to give an amide. The obtained amide was dissolved in methanol (40 ml), and sodium methoxide (2.03 mg, 37.6 mmol) was added. After stirring at room temperature for 5 hr, the mixture was concentrated, neutralized with 1N hydrochloric acid, and extracted with ethyl acetate. The organic layer was washed with brine, dried over magnesium sulfate, and concentrated under reduced pressure. The residue was purified by NH-silica gel (manufactured by Fuji Silysia Chemical Ltd.) column chromatography (eluate; ethyl acetate) to give the title compound (1.59 g, 63%) in a pale-yellow amorphous form. ^{I}H-NMR(CDCl₃) δ: 2.05 (3H, s), 3.56 (2H, s), 3.82 (3H, s), 3.89 (2H, s), 5.34 (2H, brs), 6.91 (2H, t, J = 8.0 Hz), 7.1-7.45 (9H, m), 7.56 (2H, d, J = 8.8 Hz), 7.65 (1H, s), 7.75 (2H, d, J = 8.8 Hz), 7.91 (1H, dt, J = 2.0, 7.7 Hz), 8.7-8.75 (1H, m).
elemental analysis for C₃₅H₃₀F₂N₆O₄S₂
Calculated: C, 62.86; H, 4.52; N, 12.57.
Found: C, 62.72; H, 4.31; N, 12.40.
mp 179-182°C.

### Reference Example 14

### Production of N-(4-(1-(2,6-difluorobenzyl)-5-((methylamino)methyl)-2,4-dioxo-3-(2-pyridinyl)-1,2,3,4-tetrahydrothieno[2,3-d]pyrimidin-6-yl)phenyl)-N'-methoxyurea

To a solution of the compound (1.59 g, 2.38 mmol) of Reference Example 13 in ethanol (40 ml) were added 1N hydrochloric acid (7 ml) and 10% palladium/carbon containing 50% water (0.63 g), and the mixture was vigorously stirred for 20 hr under a hydrogen atmosphere. The filtrate free of the catalyst was neutralized with 1N aqueous sodium hydroxide solution, after which the solvent was evaporated. The obtained residue was partitioned between ethyl acetate/water, and the organic layer was washed with saturated brine, and dried over anhydrous magnesium sulfate. Under reduced pressure, the solvent was evaporated, and the obtained powder was washed with diethyl ether to give the title compound (980 mg, 71%) as a pale-yellow powder.
¹H-NMR(CDCl₃) δ: 2.34 (3H, s), 3.78 (2H, s), 3.82 (2H, s), 5.38 (2H, brs), 6.92 (2H, t, J = 8.2 Hz), 7.2-7.8 (9H, m), 7.92 (1H, dt, J = 1.8 Hz, 7.6 Hz), 8.72 (1H, d, J = 4.8 Hz).

### Reference Example 15

### Production of N-(4-(1-(2,6-difluorobenzyl)-5-(((2-ethoxyethyl)(methyl)amino)methyl)-2,4-dioxo-3-(2-pyridinyl)-1,2,3,4-tetrahydrothieno[2,3-d]pyrimidin-6-yl)phenyl)-N'-methoxyurea

To a solution (4.3 ml) of the compound (251 mg) of Reference Example 14 in DMF were added 2-ethoxyethylchloride (141 mg), N-ethyldiisopropylamine (245 µl) and potassium iodide (107 mg), and the mixture was stirred at 60°C for 24 hr. The reaction mixture was partitioned between ethyl acetate/water, and the organic layer was washed with saturated brine, and dried over anhydrous sodium sulfate. The residue obtained by evaporating the solvent was purified by aminopropylsilica gel (manufactured by Fuji Silysia Chemical Ltd.) chromatography (45 g; developer; ethyl acetate/hexane:3/2→4/1), and recrystallized from ethyl acetate to give the title compound (62 mg) as colorless crystals.
elemental analysis for C₃₂H₃₂N₆O₅SF₂·0.1AcOEt
Calculated: C, 59.01; H, 5.01; N, 12.74.
Found: C, 59.11; H, 5.13; N, 12.55.
¹H-NMR (CDCl₃) δ: 1.13 (3H, t, J = 6.9 Hz), 2.15 (3H, s), 2.63 (2H, t, J = 6.2 Hz), 3.39 (2H, q, J = 6.9 Hz), 3.44 (2H, t, J = 6.2 Hz), 3.80 (2H, brs), 3.81 (3H, s), 5.34 (2H, brs), 6.91 (2H, t, J = 8.1 Hz), 7.19 (1H, s), 7.27-7.32 (1H, m), 7.35-7.41 (2H, m), 7.53 (2H, d, J = 8.4 Hz), 7.63 (1H, s), 7.64 (2H, d, J = 8.4 Hz), 7.88 (1H, dt, J = 1.2 Hz, 7.5 Hz), 8.68 (1H, dt, J = 0.9 Hz, 4.8 Hz). IR (KBr): 1717, 1674, 1591, 1530, 1460, 1329 9 cm⁻¹.

### Example 5

Using the compound (100 mg) produced in Reference Example 12, lactose (165 mg), corn starch (25 mg), polyvinyl alcohol (4 mg) and magnesium stearate (1 mg), a tablet is produced according to a conventional method.

### Example 6

Using the compound (100 mg) produced in Reference Example 15, lactose (165 mg), corn starch (25 mg), polyvinyl alcohol (4 mg) and magnesium stearate (1 mg), a tablet is produced according to a conventional method.

### Example 7

Using the compound (1 g) produced in Reference Example 12, lactose (197 g), corn starch (50 g) and magnesium stearate (2 g), a tablet is produced according to a conventional method.

### Example 8

Using the compound (1 g) produced in Reference Example 15, lactose (197 g), corn starch (50 g) and magnesium stearate (2 g), a tablet is produced according to a conventional method.

### Industrial Applicability

The premature ovulation inhibitor for use in in vitro fertilization or embryo transfer process of the present invention, which comprises a nonpeptidic compound having a gonadotropin releasing hormone antagonistic action, is low toxic, permits oral administration, and has a superior inhibitory effect on premature ovulation in in vitro fertilization or embryo transfer process. That is, the premature ovulation inhibitor for use in in vitro fertilization or embryo transfer process of the present invention, which comprises a nonpeptidic compound having a gonadotropin releasing hormone antagonistic action, has a superior GnRH antagonistic action, is low toxic, and is superior in oral absorbability, action sustainability, stability and pharmacokinetics. Thus, it can be safely used for promoting or assisting in vitro fertilization or embryo transfer process. In addition, production of the premature ovulation inhibitor of the present invention is easy and simple.

## Claims

1. A compound, N-(4-(1-(2,6-difluorobenzyl)-5-((dimethylamino)methyl)-3-(6-methoxy-3-pyridazinyl)-2,4-dioxo-1,2,3,4-tetrahydrothieno[2,3-d]pyrimidin-6-yl)phenyl)-N'-methoxyurea or a salt thereof, for use in inhibiting premature ovulation during in vitro fertilization or embryo transfer process in a mammal, excluding industrial or commercial human embryo transfer processes.

## Patentansprüche

1. Verbindung N-(4-(1-(2,6-Difluorbenzyl)-5-((dimethylamino)methyl)-3-(6-methoxy-3-pyridazinyl)-2,4-dioxo-1,2,3,4-tetrahydrothieno[2,3-d]pyrimidin-6-yl)phenyl)-N'-methoxyharnstoff oder ein Salz davon zur Verwendung bei der Hemmung einer vorzeitigen Ovulation während der in-vitro-Fertilisation oder dem Embryoübertragungsvorgang bei einem Säuger, wobei gewerbliche oder kommerzielle Übertragungsvorgänge mit menschlichen Embryonen ausgeschlossen sind.

## Revendications

1. Composé, qui est la N-(4-{1-(2,6-difluoro-benzyl)-5-[(diméthyl-amino)-méthyl]-3-(6-méthoxy-pyridazin-3-yl)-2,4-dioxo-1,2,3,4-tétrahydro-thiéno[2,3-d]pyrimidin-6-yl}-phényl)-N'-méthoxy-urée ou l'un de ses sels, pour utilisation dans le but d'inhiber une ovulation prématurée pendant un processus de fécondation in vitro ou de transplantation d'embryon chez un mammifère, à l'exception des processus industriels ou commerciaux de transplantation d'embryon humain.
